Europäisches Patentamt

European Patent Office

Office européen des brevets

(19)

(11) Publication number: **0 284 362**
A2

(12)

# EUROPEAN PATENT APPLICATION

(21) Application number: 88302537.1

(22) Date of filing: 23.03.88

(51) Int. Cl.⁴: **C 12 Q 1/68**
C 07 H 21/00, C 12 P 19/34,
C 07 K 7/10
// G01N33/574, A61K39/395

(30) Priority: 23.03.87 US 29456

(43) Date of publication of application:
28.09.88 Bulletin 88/39

(84) Designated Contracting States:
AT BE CH DE ES FR GB GR IT LI LU NL SE

(71) Applicant: IMPERIAL CHEMICAL INDUSTRIES PLC
Imperial Chemical House Millbank
London SW1P 3JF (GB)

(72) Inventor: Elvin, Paul
17 Whitecraigs Place Summerston
Glasgow G23 5LU (GB)

(74) Representative: Mack, John Richard et al
Imperial Chemical Industries PLC Legal Department:
Patents PO Box 6 Bessemer Road
Welwyn Garden City Hertfordshire AL7 1HD (GB)

The microorganism(s) has (have) been deposited with National Collection of Industrial Bacteria under number(s) NCIB 12429.

(54) **Molecular markers.**

(57) The invention relates to polynucleotide sequences and proteins that are differentially expressed during malignant tumour progression and metastasis in colorectal cancer and which may serve as general molecular markers in primary and metastatic neoplastic disease especially colorectal cancer and which may additionally provide a basis for therapy.

EP 0 284 362 A2

**Description**

## MOLECULAR MARKERS

The present invention relates to markers associated with malignant tumours such as breast tumours but especially colorectal tumours and metastases as well as to the use of such markers in the diagnosis and treatment of a range of neoplastic conditions and predispositions thereto. Such markers may, for example, take the form of genomic DNA sequences, RNA sequences obtainable from said DNA sequences, cDNA sequences obtainable from said RNA sequences or fragments of any such sequences as well as polypeptides defined by the coding portions of such sequences and antibodies obtainable by immunisation with any such polypeptide or polypeptide fragment.

One of the major causes of failure in the treatment of colorectal cancer is the occurrence of metastatic disease, involving primarily the liver and lungs, and occasionally bone. By the time of first presentation, between 15-25% of patients have metastases to the liver (Welch and Donaldson, 1987), while it has been more recently shown that about 30% of patients undergoing apparently curative resection for colorectal cancer possess occult hepatic metastases (Finley and McArdle, 1982). Furthermore, in their study of occult metastatic disease using computerised tomography (Finley and McArdle, 1982) it was shown that the presence or absence of metastatic disease at the time of clinical presentation is the most critical prognostic factor, accounting almost entirely for the observed pattern of mortality. The identification of those patients with occult metastatic disease is clearly of considerable importance in planning therapy and, in addition, would avoid the unnecessary further treatment of that group of patients surgically cured of their disease (Taylor et al, 1985).

The aggressiveness of colorectal tumours is currently assessed using the Dukes classification (Dukes, 1932). However, this, together with other prognostic indicators such as tumour morphology and serum levels of carcinoembryonic antigen (CEA), do not reliably correlate with clinical outcome (Finley and McArdle, 1982; Lewi et al, 1984). Measurement of DNA distribution patterns in tumour cell nuclei suggest that tumour ploidy may be of prognostic value, non-diploid tumours tending to be more aggressive (Wolley et al, 1982). The few clinically applicable markers that exist for colorectal cancer are of little specificity either for the diagnosis or for monitoring the course of the disease (Schwartz, 1980), although it has been suggested that serum levels of CEA (Tate, 1982) and alkaline phosphatase (Asbo et al, 1986) may be used as indicators of recurrence and of secondary disease. However, these markers do not allow a distinction between recurrent local and metastatic disease (Hine and Dykes, 1984).

Although many features of tumour cells have been studied in relation to metastasis (reviewed in Weiss, 1985), as yet no single variable has been consistently identified as being associated with the metastatic phenotype and there is no clinically reliable means of predicting the metastatic potential of a tumour. Metastasis is considered to be a multistep process (reviewed in Hart and Fidler, 1980; Nicolson, 1982; Schirrmacher, 1985) involving many phenotypic characteristics expressed as a consequence of the activity of many gene loci, and would be expected to be reflected in changes in the relative abundances of specific mRNAs.

Variations in abundance of individual mRNAs can be studied by the application of molecular cloning techniques which allow the identification of previously uncharacterised genes that are associated with a particular cell phenotype. Cloned complementary DNAs (cDNAs) representing specific abundant mRNAs have been used, for example, to identify sequences associated with normal development (Sim et al, 1979), transformation (Augenlicht and Kobrin, 1982), and to identify further markers to supplement those used in classification of leukaemias (Weidemann et al, 1983; Warnock et al, 1985; Mars et al, 1985).

The present invention is based at least in part on the discovery of nucleotide sequences and proteins that are differentially expressed during malignant tumour progression and metastasis for example in breast cancer, but especially in colorectal cancer and which may thus serve inter alia as general molecular markers in primary and metastatic neoplastic disease for example in breast cancer, and especially in colorectal cancer, as well as providing a basis for therapy. The present invention also relates to methods of detecting such markers.

Thus according to one feature of the present invention there is provided a polynucleotide sequence which is differentially expressed during malignant tumour progression and metastasis in colorectal cancer and fragments thereof.

Such polynucleotide sequences will in general be in isolated or cloned form and will include genomic DNA sequences and corresponding RNA sequences as well as cDNA sequences derived from the aforementioned RNA sequences and any fragment of such sequences. Such cDNA sequences may for example be obtained from RNA by the use of reverse transcriptase.

Such genomic DNA sequences and corresponding RNA sequences are preferably in substantially pure form.

The fragments of the aforementioned polynucleotide sequences and cDNA sequences will in general consist of at least 8 consecutive nucleotides, preferably at least 10, more preferably at least 12 , nucleotides of the polynucleotide sequence.

The polynucleotide sequences of the present invention are preferably characterised in that the cDNA sequences corresponding thereto contain the following sequence:

2

5'—AGCGTGGGTA TCGAGGCGGA CGACGACCGG CTCAACAAGG TTATCAGTGA GCTGAATGGA

AAAAACATTG AAGACGTCAT TGCCCAGGGT ATTGGCAAGC TTGCCAGTGT ACCTGCTGGT

GGGGCTGTAG CCGTCTCTGC TGCCCCAGGC TCTGCAGCCC CTGCTGCTG GTTCTGCCCC I

TGCTGCAGCA GAGGAGAAGA CAGATGAGAA GAAGGAGGAG TCTGAAGAGT CAGATGATGA

CATGGGGATT TGCCTTTTTG ATTAAATTCC TGCTCCCCTG CAATAACCTT TTTACACATC

TTA—3' end

It will be appreciated that the fragment of formula I of the present invention may be used to identify mRNA species corresponding thereto in Northern blot analyses.

The polynucleotide sequences of the present invention may also be characterised in that a cDNA sequence derived from said polynucleotide sequences comprises the sequence insert in pLM59 (NCIB No. 12429).

pLM59 has been deposited under the Budapest Treaty with The National Collections of Industrial & Marine Bacteria Ltd, Torry Research Station, PO Box No. 31, 135 Abbey Road, Aberdeen, AB9 8DG, Scotland under the deposition number NCIB 12429. The deposition date in respect of pLM59 is 19th March 1987. pLM59 consists of transformed E.coli JM83 (ATCC NO 35607) See Gene 19, p259-268, 1982 and 25 p241-247, 1983. The insert sequence in pLM59 maybe defined by EcoRI and BamHI restriction sites at terminal ends and by the length of the fragment (see Table 2)

It will be appreciated that the insert sequence in pLM59 or the polynucleotide sequence of formula I hereinbefore defined or a fragment of such sequences having at least 8, preferably at least 10, more preferably at least 12 especially at least 14 consecutive nucleotides may be used to obtain the corresponding genomic DNA or RNA sequences of humans and animals after such sequences have been closed in appropriate vectors using standard techniques known in the art (see for example T Maniatis et al; Molecular Cloning A Laboratory Manual, Cold Spring Harbor Laboratory, 1982). Polynucleotide sequences of the present invention may also be prepared by direct chemical or enzymatic synthesis or microbiological reproduction.

It will also be appreciated that polynucleotide sequences of the present invention define the sequences of polypeptides which are encoded therein. The expression of such polypeptides may itself constitute a useful marker in the investigation of malignant disease. Such polypeptides may have some biological role in the development of malignant disease and interference with this function may be useful in therapy of malignant disease.

It will be appreciated that the aforementioned molecular markers may be determined in a number of different ways. Thus for example polynucleotide probes may be constructed which are capable of hybridisation to any portion of the genomic DNA precursor of the aforesaid RNA sequence including introns and non-coding as well as coding portions of the DNA sequence.

Polynucleotide probes may also if desired be constructed which are capable of hybridisation to any portion of the aforesaid RNA sequence, regardless of whether the portion is capable of translation into a polypeptide or not. Moreover, if desired the molecular marker in the form of an RNA sequence may be transcribed into a corresponding cDNA sequence using for example reverse transcriptase and the molecular marker determined by the use of a polynucleotide probe capable of hybridising to any portion of the cDNA sequence. It will be appreciated that the polynucleotide probe will compromise a nucleotide sequence capable of hybridisation to a sufficient length of the sequence to be determined to ensure that the probe unambiguously detects the sequence of interest. In general the probe will be capable of hybridising to at least 8 consecutive nucleotides of the sequence to be determined, preferably to at least 10 consecutive nucleotides, more preferably to at least 12 consecutive nucleotides and especially to at least 14 consecutive nucleotides.

Thus according to one feature of the present invention there is provided a polynucleotide probe which comprises a nucleotide sequence capable of hybridising to a polynucleotide of the present invention or portion thereof said probe optionally having a labelled or marker component.

As stated above the polynucleotide probes of the present invention will in general be capable of hybridising to at least 8 consecutive nucleotides of the polynucleotides of the present invention, preferably at least 10 consecutive nucleotides, more preferably to at least 12 consecutive nucleotides and especially to at least 14 consecutive nucleotides.

The polynucleotide probes of the present invention may be labelled or marked according to techniques known in the art, for example, $^{32}$P-radiolabelled in any conventional way, or alternatively radiolabelled by other means well known in the hybridisation art for example to give $^{35}$S-radiolabelled probes. The probes may for example carry fluorescent markers. They may alternatively be labelled with biotin or a similar species by the method of D C Ward et al, as described in Proceedings of the 1981 ICN-UCLA Symposium on Development Biology using Purified Genes held in Keystone, Colorado on March 15-20, 1981 vol. XXIII 1981 pages 647-658 Academic Press; Editor Donald D Brown et al or even enzyme-labelled by the method of A D B Malcolm et al, Abstracts of the 604th Biochemical Society Meeting, Cambridge, England (meeting of 1 July 1983).

The aforementioned molecular markers may also be determined by the use of antibodies, which may be polyclonal but are preferably monoclonal, raised to a polypeptide sequence coded for by at least a portion of the aforementioned genomic DNA sequence or corresponding RNA sequence. The antibody may thus bind to the protein encoded by the aforementioned genomic DNA sequence or corresponding RNA sequences or bind to any fragment of the protein.

Thus according to a further feature of the present invention there is provided an antibody effective to bind at least a fragment of the polypeptide encoded by the polynucleotide of the present invention. The term "antibody" as used herein includes all Immunoglobulins and fragments thereof which contain recognition sites for antigenic determinants of polypeptides of the present invention.

The antibody of the present invention may if desired carry a label or marker component for example as hereinbefore described in relation to the polynucleotide probes of the present invention. Thus the antibodies may for example carry a fluorescent marker. It is not however necessary that the antibody of the present invention carry a label or marker component. Thus for example the antibody of the present invention may be detected by a second antibody which is an antibody to antibodies of the species of the antibodies of the present invention for example goat antimouse immunoglobulin. The second antibody will have a labelled or marker component.

The polynucleotides, polynucleotide probes polypeptides and antibodies of the present invention may find use in the following areas:-

1) Serological diagnosis - for example testing patients, e.g. predisposed to malignancies, for the presence of the protein (encoded by the polynucleotides of the present invention) or antibodies thereto in blood, urine or other body fluids, tissue or excretion products;

2) Immunohistochemistry applications - for the diagnosis of malignant disease in tissue samples

3) Diagnostic Imaging - in which case the antibody or probe will have an appropriate label or marker, for example a radioactive label or marker;

4) Therapy - a) for example antibodies of the present invention may form part of an immunotoxin, sometimes termed the "magic bullet", in order to deliver toxic agents of drugs such as plant toxins e.g. ricin preferentially to the site of a malignant or even benign tumour (see for example European Patent Application No. 84304801.8 - Publication No. 0145111);

b) for example the antibodies of the present invention may be useful as a thereapeutic;

c) for example polynucleotides of the present invention may be useful alone in therapy as anti sense DNA or RNA. Thus polynuleotides of the present invention, optionally in a vector or in a polynucleotide analogue, which contains sequences complementary to DNA or RNA defining a protein which is differentially expressed during cancer progression and metastasis or portion thereof may be employed to prevent expression of the said protein;

5) Histological analysis - polynucleotide probes (DNA or RNA) having an appropriate label or marker may be useful in in situ hybridisation for histological analysis.

6) Determination of predisposition to genetic disease -for example the polynucleotide of the present invention (DNA or RNA) may be useful in the analysis of restriction enzyme fragment length or other polymorphisms associated with a predisposition to malignant disease. Furthermore, detection of mutations within the polynucleotide sequences of the present invention in individuals may correlate with a predisposition to malignant disease.

Whilst the nucleotide sequences of the present invention were originally identified as being differentially expressed during malignant tumour progression and metastases in colorectal cancer, the nucleotide sequences have been found to be additionally associated with malignant breast disease and metastases. The polynucleotide sequences of the present invention and fragments thereof, the polypeptides of the present invention and fragments thereof and the antibodies of the present invention are thus of interest as general markers in primary and metastatic neoplastic disease and not only in relation to malignant breast disease and colorectal cancer and metastases associated therewith.

Brief description of the drawings

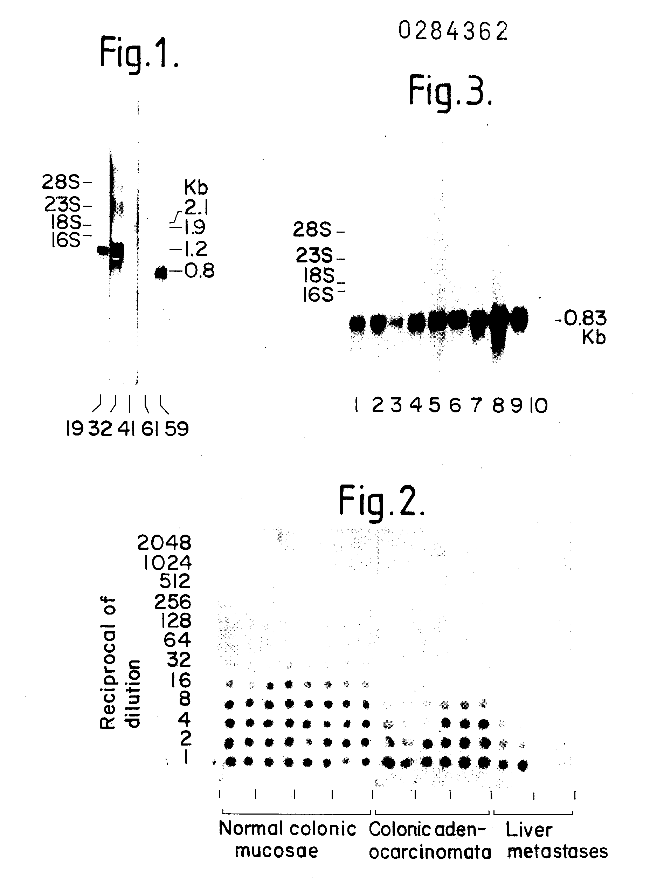

Figure 1 shows Northern blot analyses of normal mucosa RNA. Total RNA (10 µg per lane) from a sample of normal colonic mucosa was electrophoretically fractionated on a 1% agarose-formaldehyde gel and transferred to nitrocellulose. Individual lanes were hybridised with labelled recombinant plasmid probes pNM19, pNM32, pNM41, pNM61 and pLM59 as indicated (19, 32, 41, 61, 59 respectively).

Figure 2 shows Northern blot analyses of RNAs from mucosae, primary colon tumours and liver metastases. Total RNA (10 µg per lane) was electrophoretically fractionated on a 1% agarose-formaldehyde gel, transferred to nitrocellulose and hybridised with $^{32}$P-labelled recombinant pLM59 DNA. RNAs were from: lanes 1-4, normal colonic mucosae; lanes 5-7, primary tumours; lanes 8 and 9, liver metastases; lane 10, normal human liver. RNAs in lanes 1, 2 and 7 were prepared from tissue samples obtained from patients with confirmed metastatic disease, RNAs in lanes 3-6 were prepared from tissue samples obtained from patients with no evidence of secondary disease at the time of surgery.

Figure 3 shows the relative abundance of pNM32 RNA at different stages in colorectal tumour progression. The relative abundance of RNA homologous to recombinant plasmid pNM32 in tissue specimens representing different stages of colorectal tumour progression was determined by doubling-dilution RNA dot-blot hybridisation to $^{32}$p-labelled plasmid DNA. Total RNAs at a concentration of 500 µg/ml were diluted and applied to nitrocellulose as described (see Materials and Methods) in a volume of 4 µl, the first dot in each series thus representing 2µg of total RNA.

It may be advantageous to present the polynucleotide probes and/or antibodies of the present invention in the form of diagnostic kits and kits are regarded as further features of the present invention.

Thus according to a further feature of the present invention there is provided a kit for detecting polynucleotides of the present invention which comprises a polynucleotide probe as hereinbefore defined, optionally in labelled or marked form. The kit may additionally contain means for labelling or marking the probes either prior to or subsequent to hybridisation, where such probes are not already labelled or marked. The kit may also contain means for detecting said label or marker. If desired the kit may contain enzymes such as DNA polymerase or enzymes for introducing appropriately labelled nucleotides into DNA or RNA probes. The kit may contain restiction endonucleases and other appropriate materials for performing analyses of RFLP's.

According to a further feature of the present invention there is provided a kit for detecting the polypeptide of the present invention or fragments thereof which comprises an antibody of the present invention as hereinbefore defined, optionally in labelled or marked form. Where the antibody is not in labelled or marked form the kit may contain a second antibody which is an antibody to antibodies of the same species as the unlabelled antibody as hereinbefore described. The second antibody will be labelled or marked and the kit may include a format appropriate for effecting the determination for example as described in US Patent No. 4,376,110. The kit may also contain apparatus for the preparation of histological samples for analysis using antibodies of the present invention.

If desired the polypeptide of the present invention or fragments thereof may be useful as standards in analysis of samples by physical techniques, for example HPLC, TLC or other chromatographic and/or spectroscopic techniques.

According to a futher feature of the present invention there is provided a kit for manufacturing the polynucleotide of the present invention which kit comprises microorganisms containing vectors capable of producing the polynucleotides of the present invention.

For diagnostic imaging the polynucleotide probe or antibody of the present invention will have an appropriate labelled or marker component, for example a radioactive label or marker, and will conveniently be presented in a form suitable for ingestion or injection.

The antibodies of the present invention may also be of interest in purifying a polypeptide of the present invention and accordingly we further provide a method of purifying a polypeptide of the present invention as hereinbefore defined or any portion thereof or a metabolite or degradation product thereof which method comprises the use of an antibody of the present invention.

The purification method of the present invention may be effected by any convenient technique known in the art for example by providing the antibody on a support and contacting the antibody with a solution containing the polypeptide whereby the antibody binds to the polypeptide of the present invention. The polypeptide may be released from binding with the antibody by known methods for example by changing the ionic strength of the solution in contact with the complex of the polypeptide/antibody.

The following non-limiting Example is provided in order to illustrate the present invention:-

EXAMPLE 1

## MATERIALS AND METHODS

### Tissues

Specimens of histologically confirmed adenomatous polyps, colorectal tumours, and liver metastases from colorectal tumours were obtained from patients undergoing surgery at Glasgow Royal Infirmary. Specimens of histologically normal colonic mucosae were obtained from tissue adjacent to the resection margins of surgically removed colorectal tumours. All tissues were immediately frozen in liquid nitrogen and stored at -70° C until required.

### Isolation of total RNA

Total RNA was isolated from the frozen tissue specimens by a modification of the method of Chirgwin et al (1979), which yields undegraded total RNA suitable for the isolation of poly(A)$^+$ RNA, overcoming the high level of activity associated with endogenous RNAases in these tissues. A sample (about 0.51g)of the tissue specimen was ground to a fine powder under liguid nitrogen in a pre-cooled porcelain mortar and pestle. The ground tissue was lysed by transfer to 20 ml of guanidinium thiocyanate solution (5 M guanidinium thiocyanate, 5% mercaptoethanol, 50 mM tris-HCl, 50 mM EDTA, pH 7.0). DNA was fragmented by sonication, 1/10 vol 20% sarcosine added and the solution warmed to 55° C in a water bath for two minutes. Gross tissue debris was removed by centrifugation at 1000 rev/min for 10 minutes in a MSE 4L centrifuge. The solution was layered over a cushion of CsCl (5.7 M CsCl, 50 mM EDTA, pH 7.0; refractive index 1.3995) and centrifuged at 22,000 rev/min (60 000 $g_{av}$), at 17°C for 48 hours in an IEC SB-110 rotor.

The pellets were resuspended in sterile water and precipitated by adding 1/10 vol 3 M sodium acetate and 3 vol absolute ethanol. The solution was kept at -20°C overnight, and the precipitated material recovered by centrifugation at 10,000 rev/min (8700 $g_{av}$) at 4°C for 20 minutes in a Sorvall HB4 rotor. The pellets were washed in 70% and 95% ethanol and finally resuspended in sterile water at a concentration of approx. 1 mg/ml.

Poly(A)[+] RNAs were isolated from total RNAs by the method of Aviv and Leder (1972) using oligo(dT)-cellulose (BRL), recovered by precipitation and washed as described above, and finally resuspended in sterile water at a concentration of 250 μg/ml and stored at -20°C.

## cDNA library construction

Double-stranded cDNAs were synthesised from poly(A)[+] RNAs by the method of Wickens et al (1978). Oligo(dT)-primed poly(A)[+] RNA was reverse transcribed by AMV reverse transcriptase (Bio-Rad Laboratories) to generate a first strand with a hairpin loop which was used to prime second strand synthesis by E.coli DNA polymerase I (Boehringer). The hairpin loop was removed by digestion with S1 nuclease and the resultant cDNA was blunt-end ligated into the Sma1 site of plasmid pUC8. The recombinant plasmids were used to transform E.coli JM83 and individual recombinant clones were grown on L-agar 9 cm plates. Individual colonies were picked, inoculated, and grown in 96-well microtitre plates (Flow Laboratories), duplicated and stored at -20°C. Simultaneously, using a transfer plate (Dynatech), two nylon filter (Biodyne A, PALL) replicas of each plate were copied, and the bacterial DNA lysed and baked onto the filters for screening. The insert is recovered by digestion of the recombinant plasmid with Eco RI and Bam HI by methods known in the art.

## cDNA probe preparation and colony hybridisation

All of the probes used to screen the libraries were single-stranded cDNAs synthesised from poly(A)[+] RNAs using AMV reverse transcriptase (Bio-Rad Laboratories) and [32]p-dCTP (α-[32]p-dCTP, 400 Ci/mmol, Amersham International plc) as label. Colony hybridisation (Grunstein and Hogness, 1975) to the nylon filter replicas of the cDNA libraries was carried out as described by the manufacturer (PALL) at 65°C for at least 12 hours using a probe concentration of 0.5-1x10⁶ cpm/ml. Excess probe was removed by three half-hour washes in a washing buffer (5 mM $NaH_2PO_4$, 1 mM EDTA, 0.2% SDS) at 65°C. Colony hybridisation was visualised by autoradiography at -70°C using Kodak X-Omat film and Dupont Lightening Plus intensifying screens.

## Plasmid DNA isolation and dot-blot hybridisation

Small-scale bacterial cultures (2 ml overnight cultures) were used for the isolation of plasmid DNA by the method of Birnboim and Doly (1979). The DNAs were dot blotted onto Biodyne A nylon membrane filters, denatured and baked as described by the manufacturers (PALL) prior to hybridisation under conditions as described for colony hybridisation. For further study plasmids were isolated from 500 ml overnight cultures, using the alkaline lysis method of Birnboim and Doly (1979). The plasmids were purified by CsCl and sucrose gradient centrifugation. Recombinant plasmids were finally resuspended in TE buffer (10 mM tris-HCl, 1 mM EDTA, pH 8.0) at a concentration of 250 μg/ml and stored at 4°C.

## Northern blot analysis and dot-blot analysis of total RNA

Total and poly(A)[+] RNAs in a buffer solution containing 50% formamide and 2.2 M formaldehyde were heated to 65°C for 10 minutes, chilled on ice, and electrophoretically fractionated on 1% agarose-formaldehyde gels prior to Northern blotting onto nitrocellulose as described by Thomas (1980).

Serial doubling dilutions of total RNAs in sterile water were heated to 65°C for 15 minutes and chilled on ice before dot blotting onto nitrocellulose that had been previously wetted in 20X SSC (2 M NaCl, 0.3 M sodium citrate, pH 7.0) and air dried. The RNAs were immobilised onto the nitrocellulose by baking for 2 hours at 80°C.

## Southern blot analysis

Restriction enzyme digested normal human white blood cell DNA, 18 μg per lane, was electrophoretically fractionated overnight on 1% agarose gels, and then transferred to nitrocellulose using a modification of the method of Southern (1975).

## Hybridisation conditions

Recombinant plasmids were radioactively labelled by nick-translation using [32]p-dCTP (α-[32]p-dCTP, 400 Ci/mmol, Amersham International plc). Nitrocellulose filters were pre-hybridised in a buffer containing 50% formamide, 0.1% SDS, 5X Denhardt's (0.1% ficol 400K MW, 0.1% polyvinyl pyrolidine 360K MW, 0.1% bovine serum albumin), 5X SSC, 50 mM sodium phosphate , 500 μg/ml salmon sperm DNA, 10 μg/ml each of poly(A) and poly(C), 1% glycine, pH 7.0, for at least 12 hours at 42°C. Hybridisations were carried out in a buffer containing 50% formamide, 10% dextran sulphate, 0.1% SDS, 5X SSC, 1X Denhardts, 20 mM sodium phosphate, 100 μg/ml each of poly(A) and poly(C), pH 7.0, for at least 12 hours at 42°C with a probe concentration of 0.5-1x10⁶ cpm/ml. Following hybridisations filters were washed at 65°C in 2X SSC, 0.1% SDS, then 0.5X SSC, 0.1% SDS and finally 0.1X SSC, 0.1% SDS, and exposed to Kodak X-Omat film with intensifying screens at -70°C.

## RESULTS

### Screening of cDNA libraries

A cDNA library of approx. 5000 clones representative of normal colonic mucosa poly(A)$^+$ RNAs was screened with probes generated from poly(A)$^+$ RNAs according to the scheme outlined in the screening protocol set out below.

Screening protocol for cDNA libraries: Identification of
recombinants associated with tumour stage.

```
    _____        _____
    |                         |        |                         |
    |  Normal colonic mucosa  |        |  Liver metastasis        |
    |      cDNA library       |        |    cDNA library          |
    |      about 5000 clones   |        |    about 3000 clones     |
    |_____|        |_____|
                 |                                   |
Colony hybridisation 1|                              |
                 |                                   |
    probes[a]: normal mucosae(4)[b]    probes: liver metastases(2)
                 |                             colorectal ca(4)
                 |                                   |
              912 clones                          288 clones
                 |                                   |
                 |                                   |
Colony hybridisation 2|                              |
                 |                                   |
    probes:   normal mucosae(2)        probes:  normal mucosae(3)
              colorectal ca(5)                  colorectal ca(3)
                 |                               liver metastases(3)
                 |                               normal liver(1)
                 |                                   |
              89 clones                           82 clones
                 |                                   |
                 |                                   |
Plasmid DNA dot-blot|                                |
hybridisation        |                               |
                 |                                   |
    probes:   normal mucosae(3)        probes:  normal mucosae(3)
              colorectal ca(3)                  colorectal ca(3)
              liver metastases(3)              liver metastases(3)
                                                normal liver(1)
```

a. All probes were [32]p-dCTP-labelled cDNAs reverse transcribed from poly(A)[+] RNAs.

b. Figures in brackets indicate the number of different tissue specimens used to generate cDNA probes at each stage of screening.

Initially, in order to accommodate any inter-patient variation in gene expression, the library was screened with cDNA probes transcribed from RNAs from four different normal mucosae. On this basis 912 recombinant clones representing poly(A)$^+$ RNA sequences of high and medium abundance classes were identified as being common to the mucosae RNAs. Further screening of these recombinants, firstly with cDNA probes derived from two normal mucosae specimens, to establish a base-line relative hybridisation pattern, and secondly with cDNA probes derived from five different colonic tumours, identified 89 recombinants representing abundant sequences in normal colonic mucosae which were of significantly altered abundance in colorectal tumours on the basis of differences in the intensities of the autoradiographic signals.

Since the results of Grunstein-Hogness colony screening depend not only on the degree of specific hybridisation with the probes used but also on a number of variables, such as growth of a particular clone in the microtitre plate, the reproducibility of transfer and growth of bacterial colonies on the nylon filters, and recombinant plasmid copy number, plasmid DNA was isolated as described from each of the 89 recombinants and dot blotted in duplicate onto each of four replica nylon filters. Hybridisation of plasmid DNA dot blots with cDNA probes generated from normal colonic mucosae, colonic tumours, and liver metastases of colonic tumours was carried out sequentially such that no individual filter was re-hybridised with an identical class of probe, each filter was hybridised with each of the aforementioned classes of probe, and three different tissue specimens from each histological tissue type were used.

The colony hybridisation and plasmid DNA dot blot assays identified a number of recombinant clones as being representative of RNAs associated with different stages of tumour progression. These clones are detailed in Table 1. From the normal colonic mucosa library, six clones were identified as representing sequences of considerably reduced abundance in, or absence from, secondary tumours compared to primary tumours or normal tissue. In addition, a group of seven clones also represented sequences of reduced abundance in secondary tumours compared to normal tissue and primary tumours, which were assigned to a separate group on the basis of this semi-quantitative screening. A further group of seven clones appeared to represent sequences of increased abundance in primary tumours compared to normal tissue or secondary tumours. No clones apparently representing secondary tumour-specific sequences were identified in the normal colonic mucosa library.

In order to identify sequences specifically associated with metastasis, a cDNA library of approx. 3000 clones representing the more abundant poly(A)$^+$ RNAs of a liver metastasis from a colorectal tumour was also screened as outlined in the screening protocol. Sequences common to liver metastases, but of altered abundance in primary tumours, were identified by differential screening with cDNA probes derived from two secondary and four primary colorectal tumours. Further screening of these selected clones with cDNA probes derived from liver metastases, primary tumours, normal colonic mucosae and normal human liver identified 82 clones that represented RNA sequences in metastases which were of altered abundance in primary tumours, but were absent from the local RNA isolated from normal human liver. Plasmid DNA was isolated from these recombinant clones and hybridised with three different cDNAs transcribed from RNA from each histologically graded tissue type.

This screening protocol identified two groups of clones in the liver metastasis library: a group of six clones representing sequences of increased abundance in primary tumours compared to normal colonic mucosa or secondary tumours, and a group of eight clones representing sequences of increased abundance in secondary tumours compared to normal tissue or primary tumours (Table 1).

On the basis of these semi-quantitative changes in hybridisation signal intensity, four recombinant clones from the normal colonic mucosa library and one recombinant clone from the liver metastasis library were selected for further characterisation by Northern blot and RNA dot-blot analysis.

## Characterisation of selected recombinants

Following restriction enzyme digestion of recombinant plasmids, the cDNA inserts in the recombinants pNM19, pNM32, pNM41 and pNM61 from the normal colonic mucosa library, and pLM59 from the liver metastasis library, were shown to be of between 230-530 bp by agarose gel electrophoresis. Northern blotting of total RNA from a specimen of normal colonic mucosa, followed by hybridisation with nick-translated plasmids, identified these recombinants as being homologous to RNAs of between 0.8 and 2.1 kb (Fig. 1 and Table 2). In addition, sequences homologous to the recombinant clone pLM59, which represented a RNA of increased abundance in metastases relative to normal tissue, could be detected in total RNAs from patients both with and without a clinical history of disseminated disease (Fig. 2). Southern blot analysis of EcoRI and HindIII. digested normal human white blood cell DNA (results not shown) indicated that each of these recombinants represented unique RNAs.

## Relative abundance of selected recombinant-homologous RNAs

The relative abundances of RNA sequences homologous to the five recombinants, that on the basis of prior semi-quantitative screening, were closely associated with metastases were determined in a series of tissue specimens corresponding to different stages of colorectal tumour progression (Fig. 3). Four of the recombinants, clones pNM19, pNM32, pNM41 and pNM61, were found to represent RNAs reduced 5- to 10-fold in abundance in metastases relative to primary tumours, and 10- to 14-fold in metastases relative to normal mucosae. One recombinant, clone pLM59, represented a RNA showing a 4- to 6-fold increase in abundance in metastases relative to primary tumours and normal mucosae (Tables 3 and 4). To confirm that the observed differences in abundance of sequences homologous to the cloned cDNAs were due to differences in specific hybridisation and not to errors in the estimates of the RNA content of the samples, the same dot blots were stripped and reprobed with a cloned fragment of human 18S ribosomal DNA (results not shown). RNA dot-blot analysis also revealed considerable variation in the abundance of homologous RNAs to the cloned sequences at different stages of tumour progression (Table 3). That these differences were not attributable to degradation of the RNA samples concerned was shown by hybridisation of Northern blots to skeletal muscle actin (Shani et al, 1981), and $\beta_2$-microglobulin (Suggs et al, 1981) cDNA probes (results not shown).

## DISCUSSION

Molecular cloning of cDNAs representing individual mRNAs from the total poly(A)$^+$ RNA populations of normal and neoplastic tissue of the colon has enabled us to identify changes in the abundance of specific RNAs reflecting phenotypic characteristics associated with tumour progression and metastasis.

We previously screened cDNA libraries of about 1000 recombinant clones from poly(A)$^+$ RNAs representing clinically metastasising and non-metastasising variants of colorectal tumours (Kerr et al, 1983) and, although quantitative RNA dot-blot hybridisation analysis identified cDNA clones corresponding to sequences of greater abundance in RNAs from tumours compared to normal mucosae, no clones were found that consistently distinguished between localised and disseminated disease. The present study is based upon the random cloning of cDNAs representing the steady-state levels of total poly(A)$^+$ RNAs from normal mucosa and from a liver metastasis of a colorectal carcinoma. The frequency of a single cloned sequence in the cDNA library reflects the abundance of that sequence in the original mRNA population, and is ultimately determined by the turnover of the corresponding mRNA, which in turn may depend on the parent tissue. The majority of sequences comprising the abundant and moderately abundant classes of mRNA from the 10 000 - 30 000 different sequences in typical eukaryotic tissues may be expected to be represented in a cDNA library of between 5000 and 10,000 clones (Williams, 1981). Thus the libraries screened in this study should have been large enough to contain most abundant and moderately abundant sequences, although low abundance mRNAs will not have been well represented or protected. However, in a study of tissue-related differences in mRNA populations, Hastie and Bishop (1976) concluded that the most striking differences between tissues could be found among the abundant sequences. Furthermore, differences between normal and SV40-transformed human fibroblast mRNA populations could be ascribed to a few sequences of the high abundance mRNA classes (Williams et al, 1977).

By screening two cDNA libraries we have identified a number of cDNA clones homologous to RNAs of significantly reduced or increased abundance in metastases relative to neoplastic and normal colonic tissue. Screening those recombinants representing abundant RNAs common to normal colonic tissue specimens identified sequences expressed at different levels in primary and secondary tumours. Similarly, screening those recombinants representing sequences common to liver metastases of colorectal tumours identified sequences that represented RNAs associated with colorectal neoplasia and metastasis. Although histologically normal mucosa from tumour-bearing patients may exhibit phenotypic changes associated with the disease (Shamsuddin et al, 1981), these differences should not have severely masked those specifically associated with transformation or metastasis.

On the basis of colony and plasmid DNA dot hybridisations the vast majority of cDNA clones in the two libraries were found to correspond to abundant RNA sequences shared by both normal and neoplastic colon; most of these sequences were also present in the total poly(A)$^+$ RNA of normal liver. Those sequences showing some variation in abundance associated with tumour development (representing only 0.4% of the total sequences examined) were poorly represented in the poly(A)$^+$ RNA of normal liver, suggesting that the changes in abundance that occur do so in sequences characteristic of the colon. Furthermore, the clones we have identified suggest that the development of metastatic tumour cell populations in colorectal tumours is not solely due to the aberrant expression of one or two genes, but rather to the subtle alteration of multiple genetic loci. The results, however, do not allow any distinction to be made between changes in gene expression that may be associated with the prior existence of metastatic tumour cell populations (Fidler et al, 1978) and those that may arise, for example, as a result of selected pressures exerted by the host microenvironment (Schirrmacher, 1980; Kerbel et al, 1984).

Changes in gene expression are implied in tumour progression (Foulds, 1975) and the generation of clonal diversity (Nowell, 1976) responsible for heterogeneity within tumours for a number of phenotypic characteristics, which may include metastatic capability (Fidler and Hart, 1982). However, the precise nature of the genetic events associated with tumour progression remain largely undetermined. A number of cellular oncogenes have been identified as being aberrantly expressed in colorectal cancer, including c-myc (Rothberg et al, 1985; Stewart et al, 1986) c-myb (Atlitalo et al, 1984) c-Ha-ras and c-Ki-ras (Spandidos and Kerr, 1984), but their role in colorectal tumour development is unclear. Although decreased levels of p21$^{ras}$ were demonstrate in metastases, regardless of site, compared to primary colon tumours (Gallick et al, 1985) there appears to be no correlation between the levels of ras-related cellular RNA and clinical outcome with regard to the development of metastatic disease (Kerr et al, 1986). Amplification of c-myc may be correlated with tumour metastasis (Yokota et al, 1986) and the transfection of cellular oncogenes (Thorgeirsson et al,

10

1985) has shown them to be involved in the acquisition of metastatic capability. Other, as yet uncharacterised, sequences (Berstein and Weinberg, 1985), and sequences such as those we have identified, may be related to the events associated with the activation of these and other genes during the metastatic process.

These sequences may also prove to be of considerable prognostic value in predicting the metastatic capability of primary tumours at the time of surgery.

## EXAMPLE 2

The insert sequence of formula I (as hereinbefore defined) from pLM59 was used to screen breast DNA samples to see if any alterations to the genomic sequences could be detected in any of these samples. To date 35 primary breast tumours, 10 lymph node metastases from primary breast tumours and 25 DNA samples from normal individuals (placenta or lymphocyte DNA) have been screened. Whilst amplification or rearrangement of the pLM59 genomic sequences has not been detected in any samples, an EcoRI RFLP has been detected. This RFLP (presence of a 8.5 kb fragment) appears to be more frequent in aggressive breast tumours than in normal samples although the survey size, particularly of normal females is still relatively small. 10 mother-father pairs have been studied to find an informative pair, with one parent only having both sizes of the EcoRI fragment. Two such pairs were identified, and in an extended study of all four grandparents plus four children from each family, it has been shown that the RFLP is inherited in a Mendelian manner as might be expected.

## REFERENCES

Aabo, K., Pedersen, H. and Kjaer, M. Carcinoembryonic antigen (CEA) and alkaline phosphatase in progressive colorectal cancer with special reference to patient survival. Eur. J. Cancer Clin. Oncol., 22, 211-217 (1986).

Alitalo, K., Winquist, R., Lin, C.C., De La Campelle, A., Schwab, M. and Bishop, J.M. Aberrant expression of an amplified c-myb oncogene in two cell lines from a colon carcinoma. Proc. Nat. Acad. Sci., 81, 4534-4538 (1984).

Augenlicht, L.H. and Kobrin, D. Cloning and screening of sequences expressed in a mouse colon tumour. Cancer Res., 42, 1088-1093 (1982).

Aviv, H. and Leder, P. Purification of biologically active globin mRNA by chromatography on oligothymidylic acid-cellulose. Proc. Nat. Acad. Sci., 69, 1408-1412 (1972).

Bernstein, S.C. and Weinberg, R.A. Expression of the metastatic phenotype in cells transfected with human metastatic tumour DNA. Proc. Nat. Acad. Sci., 82, 1726-1730 (1985).

Birnboim, H.C. and Doly, J. A rapid alkaline extraction procedure for screening recombinant plasmid DNA. Nuc. Acids Res., 7, 1513-1523, (1979).

Chirgwin, J.M., Przybyla, A.E., MacDonald, R.J. and Rutter, W.J. Isolation of biologically active ribonucleic acid from sources enriched in ribonuclease. Biochemistry, 18, 5294-5299 (1979).

Dukes, C.E. The classification of cancer of the rectum. J. Pathol. Bacteriol., 35, 323-332 (1932).

Fidler, I.J., Gersten, D.M. and Hart, I.R. The biology of cancer invasion and metastasis. ADV. Cancer Res., 28, 149-250 (1978).

Fidler, I.J. and Hart, I.R. Biological diversity in metastatic neoplasms: Origins and implications. Science, 217, 998-1003 (1982).

Finlay, I.G. and McArdle, C.S. The identification of patients at high risk following curative resection for colorectal carcinoma. Br. J. Surg. 69, 583-584 (1982). Foulds, L. Neoplastic development Vol. 1, Academic Press Inc., New York (1975).

Gallick, G.E., Kurzrock, R., Kloetzer, W.S., Arlinghaus, R.B. and Gutterman, J.U. Expression of p21[ras] in fresh primary and metastatic human colorectal tumours. Proc. Nat. Acad. Sci., 82, 1795-1799 (1985).

Grunstein, M. and Hogness, D. Colony hybridisation: A method for the isolation of cloned DNAs that contain a specific gene. Proc. Nat. Acad. Sci., U.S.A. 72, 3961-3965 (1975).

Hart, I.R. and Fidler, I.J. Role of organ selectivity in the determination of metastatic patterns of B16 melanoma. Cancer Res., 40, 2281-2287 (1980).

Hastle, N.D. and Bishop, J.O. The expression of three abundance classes of messenger RNA in mouse tissues. Cell, 9, 761-774 (1976).

Hine, K.R., Dykes, P.W. Serum CEA testing in the post-operative surveillance of colo-rectal cancer. Br. J. Cancer 49 689-693, 1984.

Kerbel, R.S., Frost, P., Liteplo, R., Carlow, D.A. and Elliott, B.E. Possible epigenetic mechanisms of tumour progression: Induction of high frequency heritable but phenotypically unstable changes in the tumourigenic and metastatic properties of tumour cell populations by 5-azacytidine treatment. J. Cell Physiol. Supp., 3, 87-97 (1984).

Kerr, I.B., Finlay, I.G., Birnie, G.D. and McArdle, C.S. Isolation of putative metastasis-related cDNA clones from colorectal cancer. Br. J. Surg., 70, 486 (1983).

Kerr, I.B., Spandidos, D.A., Finlay, I.G., Lee, F.D. and McArdle C.S. The relation of ras family oncogene expression to conventional staging criteria and clinical outcome in colorectal carcinoma. Br. J. Cancer 53, 231-235 (1986).

Lewi, H., Blumgart, C.H., Carter, D.C., Gillis, G.R., Hole, D., Ratcliffe, J.G., Wood, C.B., McArdle, C.S. Pre-operative carcinoembryonic antigen and survival in patients with colorectal cancer. Br. J. Surg., 71, 206-208 (1984).

Mars, W.M., Florine, D.L., Talpaz, M. and Saunders, G.F. Preferentially expressed genes in chronic myelogenous leukaemia. Blood, 65, 1218-1225 (1985).

Nicolson, G.L. Cancer metastasis: Organ colonisation and the cell surface properties of malignant cells. Biochem. Biophys. Acta, 695, 113-176 (1982).

Nowell, P.C. The clonal evolution of tumour cell populations. Science. 194, 23-28 (1976).

Rothberg, P.G., Spandorfer, J.M., Erisman, M.D., Staroscik, R.N., Sears, H.F., Petersen, R.O. and Astrin, S.M. Evidence that c-myc expression defines two genetically distinct forms of colorectal adenocarcinoma. Br. J. Cancer, 52, 629-632 (1985).

Schirrmacher, V. Shifts in tumour cell phenotypes induced by signals from the microenvironment: Relevance for the immunobiology of cancer metastasis. Immunobiol., 157, 89-98 (1980).

Schirrmacher, V. Cancer metastasis: Experimental approaches, theoretical concepts and impacts for treatment strategies. Adv. Cancer Res., 43, 1-73 (1985).

Schwartz, M.K. Markers in screening: Overview of usefulness of markers. In: Colorectal cancer: Prevention, Epidemiology and Screening, Eds. Winawer, S., Schottenenfeld, D., Sherlock. P. Raven Press, N.Y. (1980).

Shamsuddin, A.K.M. Weiss, L., Phelps, P.C. and Trump, B.F. Colon epithelium: IV. Human colon carcinogenesis: Changes in human colon mucosa adjacent to and remote from carcinomas of the colon. J. Nat. Canc. Inst., 66 , 413-419 (1981).

Shani, M., Nudel, U., Zevin-Sonkin, D., Zakut, R., Givol, D., Katcoff, D., Carmon, Y., Reiter, J., Frischauf, A.M. and Yaffe, D. Skeletal muscle actin mRNA. Characterisation of the 3' untranslated region. Nuc. Acids. Res., 9, 579-589 (1981).

Sim, G.K., Kafatos, F.C., Jones, C.W., Koehler, M.D., Efstratiadis, A., Maniatis, T. Use of a cDNA library for studies on evolution and development expression of the chorion multigene families. Cell, 18, 1303-1316 (1979).

Southern, E.M. Detection of specific sequences among DNA fragments separated by gel electrophoresis. J. Mol. Biol., 98, 503-517 (1975).

Spandidos, D.A. and Kerr, I.B. Elevated expression of the human ras oncogene family in premalignant and malignant tumours of the colorectum. Br. J. Cancer, 49, 681-688 (1984).

Stewart, J., Evan, G., Watson, J. and Sikora, K. Detection of the c-myc oncogene product in colonic polyps and carcinomas. Br. J. Cancer, 53, 1-6 (1986).

Suggs, S.V., Wallace, R.B., Hirose, T., Kawashima, E.H. and Itakura, K. Use of synthetic oligonucleotides as hybridisation probes: Isolation of cloned cDNA sequences for human $\beta_2$-microglobulin. Proc. Nat. Acad. Sci., 78, 6613-6617 (1981).

Tate, H. Plasma CEA in the post surgical monitoring of colorectal carcinoma. Br. J. Cancer, 46, 323-330 (1982).

Taylor, I., Machin, D., Mullee, M., Trotter, G., Cooke, T. and West, C. A randomised controlled trial of adjuvant portal vein cytotoxic perfusion in colorectal cancer. Br. J. Surgery, 72, 359-363 (1985).

Thomas, P.S. Hybridisation of denatured RNA and small DNA fragments transferred to nitrocellulose. Proc. Nat. Acad. Sci., 77, 5201-5205 (1980).

Thorgeirsson, U.P., Turpeenniemi-Hujanen, T., Williams, J.E. Westin, E.H., Heilman, C.A., Talmadge, J.E. and Liotta, L.A. NIH/3T3 cells transfected with human tumour DNA containing activated ras oncogenes express the metastatic phenotype in nude mice. Mol. Cell. Biol., 5, 259-262 (1985).

Warnock, A.M., Burns, J.H. and Birnie, G.D. Subdivision of the acute non-lymphoblastic leukaemias by measurement of the relative abundance of a specific RNA sequence. Leuk. Res., 9, 955-966 (1985).

Weiss, L. In Principles of metastasis, Acad. Press London 1985.

Welch, J.P. and Donaldson, G.A. Detection and treatment of recurrent cancer of the colon and rectum. Am. J. Surg., 135, 505-510 (1978).

Wickens, M.P., Buell, G.N. and Schimke, R.T. Synthesis of double stranded DNA complementary to lysozyme, ovomucoid, and ovalbumin mRNAs. J. Biol. Chem., 253, 2483-2495 (1978).

Wiedemann, L.M., Burns, J.H. and Birnie, G.D. Differences among the polyadenylated RNA sequences of human leukocyte populations: An approach to the objective classification of human leukaemias. EMBO. J., 12, 9-13 (1983).

Williams, J.G. The preparation and screening of a cDNA clonebank. In: Genetic Engineering vol. 1, 1-59 Ed. Williamson, R., Academic Press, London (1981).

Williams, J.G., Hoffman, R. and Penman, S. The extensive homology between mRNA sequences of normal and SV-40 transformed human fibroblasts. Cell, 11, 901-907 (1977).

Wolley, R.C., Schreiber, K., Koss, L.G., Karas, M. and Sherman, A. DNA distribution in human colorectal carcinomas and its relationship to clinical behaviour. J. Nat. Canc. Inst., 69, 15-22 (1982).

Yokota, J., Tsunetsugu-Yokota, Y., Battifora, H., Le Fevre, C. and Cline, M.J. Alterations of myc, myb, and ras$^{Ha}$proto-oncogenes in cancers are frequent and show clinical correlation. Science, 231, 261-265 (1986).

Table 1.    Recombinant cDNA dot blot hybridisation to cDNA probes
from histologically graded tissues

| Origin of recominant clones | Number of clones[b] | Relative hybridisation to cDNA probes representing[a] | | |
|---|---|---|---|---|
| | | Mucosa | Primary tumour | Secondary tumour |
| Normal colonic | 6 | +[c] | + | − |
| mucosa | 7 | + | + | +/− |
| cDNA library | 7 | + | ++ | + |
| Liver metastasis | 8 | + | + | ++ |
| cDNA library | 6 | + | ++ | + |

a.  $^{32}$P-labelled single-strained cDNAs reverse transcribed from
total poly(A)[+] RNA.

b.  Clones grouped on the basis of hybridisation of plasmid DNA dot
blots with probes indicated;  identical results obtained with
probes derived from three specimins of each tissue type.

c.  Hybridisation signals: ++, very strong; +, strong; −, weak or
absent.

Table 2. Characteristics of five selected cloned sequences.

| cDNA clone | Size of cDNA insert (bp) | Size of homologous RNA (kb) |
|---|---|---|
| pNM19 | 530 | 1.2 |
| pNM32 | 485 | 1.2 |
| pNM41 | 420 | 1.9 |
| pNM61 | 230 | 2.1 |
| pLM59 | 400 | 0.8 |

Table 3. Relative abundances of five mRNAs in mucosae, polyps, carcinomas and metastases.

| Tissues | Recombinant clones | | | | |
|---|---|---|---|---|---|
| | pNM19 | pNM32 | pNM41 | pNM61 | pLM59 |
| Mucosa (4)[a] | 104[b] | 320 | 98 | 130 | 5 |
| Polyp (3) | 88 | 192 | 192 | 85 | 4 |
| Carcinoma (4) | 107 | 32 | 32 | 32 | 8 |
| Metastases (2) | 10 | 24 | 9 | 9 | 32 |

a. Number of individual samples.

b. Mean values of reciprocals of dilution end—points determined by total RNA doubling—dilution dot—blot assay (see fig.4).

14

Table 4.    Abundances of homologous RNAs in metastases relative to mucosa and carcinoma.

| Abundance in metastases | Recombinant clone | | | | |
|---|---|---|---|---|---|
| | pNM19 | pNM32 | pNM41 | pNM61 | pLM59 |
| Relative to mucosa | 0.1 | 0.08 | 0.09 | 0.07 | 6.4 |
| Relative to carcinoma | 0.1 | 0.75 | 0.28 | 0.28 | 4.0 |

## Claims

1. A polynucleotide sequence which is differentially expressed during malignant tumor progression and metastasis in colorectal cancer and fragments thereof.

2. A polynucleotide sequence as claimed in claim 1 characterised in that the cDNA sequence corresponding thereto contains the sequence of formula I as herein defined or a sequence complementary thereto.

3. A polynucleotide sequence as claimed in claim 1 characterised in that a cDNA sequence derived from said polynucleotide sequences comprises the insert sequence in pLM59 (NCIB No. 12429).

4. A process for preparing a polynucleotide sequence as defined in any one of the preceding claims which comprises the use as a probe of the insert sequence in pLM59 or the polynucleotide sequence of formula I as defined in claim 2 or a fragment of such sequences having at least 8 consecutive nucleotides, to obtain the corresponding genomic DNA or RNA polynucleotide sequences as defined in any one of the preceding claims.

5. A process for preparing a cDNA polynucleotide sequence as defined in any one of claims 1 to 3 which comprises synthesising the cDNA sequence from an RNA polynucleotide sequence as defined in any one of claims 1 to 3 by enzymatic techniques known per se.

6. A process for preparing a polynucleotide sequence as defined in any one of claims 1 to 3 by chemical or enzymatic synthesis or by microbiological reproduction.

7. A polypeptide or fragment thereof, encoded by a polynucleotide sequence as defined in any one of claims 1 to 3.

8. An antibody effective to bind at least a fragment of the polypeptide as claimed in claim 7.

9. A polynucleotide probe which comprises a nucleotide sequence capable of hybridising to a polynucleotide sequence which is differentially expressed during malignant tumour progression and metastasis in colorectal cancer and fragments thereof.

10. A polynucleotide probe as claimed in claim 9 wherein the nucleotide sequence is capable of hybridising to a polynucleotide sequence as defined in claim 2 or claim 3 or a fragment thereof.

11. A method for the diagnosis or prognosis of malignant disease which comprises detecting the presence or absence in a sample of a polynucleotide or fragment thereof as defined in any one of claims 1 to 3, a polypeptide or fragment thereof as defined in claim 7 or an antibody as defined in claim 8.

12. A method of determining the presence or absence of a predisposition to malignant disease which comprises the use of a polynucleotide or fragment thereof as defined in any one of claims 1 to 3 in detecting the presence or absence of polymorphisms associated with the predisposition to malignant disease.

13. A method of determining the presence or absence of a predisposition to malignant disease which comprises detecting the presence or absence of a mutation within the polynucleotide sequence defined in any one of claims 1 to 3.

# Fig.1.

0284362

# Fig.3.

Figures showing Fig.1 (28S, 23S, 18S, 16S markers with Kb values 2.1, 1.9, 1.2, 0.8; lanes 19 32 41 61 59), Fig.3 (28S, 23S, 18S, 16S markers with 0.83 Kb band; lanes 1 2 3 4 5 6 7 8 9 10), and Fig.2 (Reciprocal of dilution values 2048, 1024, 512, 256, 128, 64, 32, 16, 8, 4, 2, 1 across Normal colonic mucosae, Colonic adenocarcinomata, Liver metastases).

# Fig.2.